# EUROPÄISCHE PATENTSCHRIFT

(11) **EP 1 066 809 B1**
(45) Veröffentlichungstag und Bekanntmachung des Hinweises auf die Patenterteilung: **10.09.2003**
(21) Anmeldenummer: 00104025.2
(22) Anmeldetag: 26.02.2000
(51) Int. Cl.: A61F 13/15

(54) **Aufnahme- und Verteilungselement für Flüssigkeiten in absorbierenden Artikeln**
Acquisition and distribution element for absorbent articles
Element d'acquisition et distribution pour des articles absorbents

(30) Priorität: 07.07.1999 DE 19931192
(43) Veröffentlichungstag der Anmeldung: 10.01.2001
(73) Patentinhaber: Sandler AG, 95126 Schwarzenbach/Saale (DE)
(72) Erfinder: Sandler, Christian Heinrich, Dr., 95126 Schwarzenbach/S. (DE); Magiera, Dieter, 95111 Rehau (DE); Höflich, Wolfgang, 95126 Schwarzenbach/S. (DE)

(56) Entgegenhaltungen:
- US-A- 5 820 973
- US-A- 5 876 388

## Beschreibung

Die Erfindung betrifft ein Aufnahme- und Verteilungselement für Flüssigkeiten in absorbierenden Artikeln, das als Komponente für Einwegartikel im Hygienebereich eingesetzt werden kann, also z.B. für Babywindeln, Damenbinden, Slipeinlagen, Erwachseneninkontinenzhilfen ("Erwachsenenwindeln") und dergleichen.

Zur Ausstattung der beispielhafte erwähnten Hygieneartikel können sich weitere Komponenten z.B. Windelverschlüsse, Klebestreifen, elastische Bänder, Beinabschlüsse, Haftsysteme etc. als nützlich erweisen. Das diese Komponenten für den Gegenstand der vorliegenden Erfindung nicht wesentlich sind, werden diese in der folgenden Beschreibung nicht näher erläutert.

In der folgenden Beschreibung wird sich der Einfachheit halber häufig auf die Anwendung des erfindungsgemäßen Aufnahme- und Verteilungselementes in einer Babywindel bezogen. Dies ändert jedoch nichts an der Tatsache, daß die weiteren, beispielhaft eingangs eingeführten Einwegartikel für den Hygienebereich im Sinne dieser Erfindung ausgebildet werden können.

Die Basisaufgabe von Einweg-Hygieneprodukten ist die Aufnahme und die Sicherung von Körperausscheidungen.
Die, diese Aufgabe erfüllende Komponente der Einweg-Hygieneprodukte ist meist schichtweise aufgebaut und besteht zumindest aus einer körperseitigen, flüssigkeitsund luftdurchlässigen Abdeckung, aus einer, der Bekleidung zugewandten flüssigkeitsundurchlässigen Abdeckung und einem zwischen diesen Schichten angeordneten flüssigkeitsspeichernden Kern.

Absorbierende Einwegartikel funktionieren, vereinfacht dargestellt, in der Weise, daß die vom Körper abgegebenen Flüssigkeiten die körperseitige, flüssigkeitsdurchlässige Abdeckung durchdringen und vom absorbierenden Kern aufgenommen und festgehalten werden. Die bekleidungsseitige Abdeckung verhindert, daß Teile der vom Kern aufgenommenen Flüssigkeit, den absorbierenden Artikel in Richtung der Kleidung des Trägers verlassen und dessen Kleidung verschmutzen.

Es ist bekannt, daß das Einbringen von Flüssigkeit in Hygieneprodukte in sehr kurzer Zeit und in relativ großer Menge erfolgen kann ("Flüssigkeitsschwall"), weshalb es erforderlich ist, daß die relativ große Flüssigkeitsmenge nahezu augenblicklich vom absorbierenden Artikel aufgenommen wird, und diese aufgenommene Flüssigkeitsmenge nicht mehr an den Körper abgegeben wird. Es muß daher verhindert werden, daß sich, beispielsweise als Folge einer zu geringen Sauggeschwindigkeit des Saugkernes, unterhalb der körperseitigen Abdeckung ein Flüssigkeitsstau bildet, von dem aus die Flüssigkeit Hygieneprodukt an unerwünschten Stellen aus dem Hygieneprodukt herauslaufen könnte.

Der flüssigkeitsspeichernde Kern moderner Hygieneprodukte enthält häufig neben einer Matrix aus hauptsächlich hydrophilen Zellulose-Pulp-Fasern einen großen Anteil superabsorbierender, hydrogelbildender Partikel, so daß in den flüssigkeitsspeichemden Kern eines Hygieneproduktes eintretende Flüssigkeit sehr intensiv gebunden werden kann. Hierbei entsteht aber das Problem, daß, wenn der Flüssigkeitsschwall überwiegend senkrecht in den flüssigkeitsspeichernden Kem eintritt, die große Flüssigkeitsmenge auf relativ kleiner Fläche verteilt wird und infolge dessen nur die genau unter dieser Fläche befindlichen superabsorbierenden Partikel zur Flüssigkeitsbindung in Aktion treten können. Die außerhalb dieser Fläche befindlichen superabsorbierenden Partikel bleiben dagegen ungenutzt. Ein großer Flüssigkeitschwall auf einer kleinen Fläche bringt die von der Flüssigkeit benetzten superabsorbierenden Partikel sehr schnell zum Quellen, wodurch sich in der Auftreffzone des Saugkerns des Hygieneproduktes eine Gelschicht bildet, welche das weitere Eindringen der Flüssigkeit in das Innere des flüssigkeitsspeichernden Kerns verhindert. Diesen Zustand bezeichnet man als Gel-Blocking". Er verhindert eine entsprechend gute Aufnahme, spontan auftretender großer Flüssigkeitsmengen, weshalb man bestrebt ist, eine solche Flüssigkeitsmenge vor dem Eindringen der Flüssigkeit in den flüssigkeitsspeichernden Kem auf eine möglichst große Fläche zu verteilen, so daß die im Saugkern befindlichen superabsorbierenden Partikel möglichst komplett ausgenutzt werden und die Flüssigkeitsmenge je Flächeneinheit möglichst gering wird, wodurch erreicht werden soll, daß sich das sog. Gel-Blocking nicht oder kaum negativ auswirkt.

Aus den vorstehenden Ausführungen kann geschlossen werden, daß es notwendig ist, zwischen der bekleidungsseitigen Abdeckung und dem flüssigkeitsspeichernden Kern zwei weitere Schichten einzubringen, nämlich eine erste Schicht, welche in der Lage ist, große auftretende Flüssigkeitsmengen schnell von der bekleidungsseitigen Abdeckung wegzuführen und ggf. so lange zwischenzuspeichem, bis der flüssigkeitsspeichernde Kem die gesamte Flüssigkeit aufgenommen und gebunden hat.
Die zweite in Frage kommende Schicht wäre eine Flüssigkeitsverteilschicht, welche die Aufgabe hat, die in der Aufnahmeschicht zwischengespeicherte Flüssigkeit auf die gesamte Fläche des flüssigkeitsspeichernden Kernes zu verteilen, so daß die gesamte Speicherkapazität des flüssigkeitsspeichernden Kerns in optimaler Weise genutzt wird, und Gelblocking vermieden wird.

Aus der DE 197 30 181 ist ein absorbierendes Erzeugnis bekannt, welches zwischen poröser Oberschicht und absorbierendem Kern eine poröse Annahmeschicht besitzt, bestehend aus durch Klebemittel verbundene Polyolefin-Fasem. Eine derartige Aufnahmeschicht ist für die Aufnahme eines großen Flüssigkeitsschwalles geeignet, ist aber nicht oder kaum in der Lage, die aufgenommene Flüssigkeit auf die Gesamtfläche des flüssigkeitsspeichernden Kernes zu verteilen, weil durch die groben Poren der porösen Annahmeschicht die Flüssigkeit diese bevorzugt in z-Richtung durchdringt, anstatt von Kapillarkräften in x-y-Richtung verteilt zu werden.

In der EP 674 891 ist eine Flüssigkeitsverteilschicht für absorbierende Artikel beschrieben, welche aus einem Mikrofaservliesstoff besteht, der in der Lage ist, in absorbierenden Artikeln eingedrungene Flüssigkeitsmengen in einer Weise über die Fläche des flüssigkeitsspeichernden Kernes zu verteilen, daß die Speicherkapazität des Kernes in optimaler Weise ausgenutzt wird. Eine derartiger Mikrofaservliesstoff ist aber aufgrund seiner naturgemäß hohen Dichte nicht in der Lage einen großen Flüssigkeitsschwall in der geforderten Weise aufzunehmen und zwischenzuspeichern.

Die US-Patentschrift 5,820,973 beschreibt ein Flüssigkeitsaufnahme- und Verteilschicht, welche zweischichtig aufgebaut ist und welche in der Lage ist, den durch die körperseitige Abdeckung gedrungenen Flüssigkeitsschwall zwischenzuspeichern, und in der dichteren zweiten Schicht die Flüssigkeitsmenge über die Fläche des flüssigkeitsspeichernden Kernes zu verteilen. Bei dieser Konstruktion kann das Problem auftreten, daß bei sehr großen anfallenden Flüssigkeitsschwällen bei zu dichter zweiter Schicht die erste Speicherschicht die Flüssigkeit über eine zu lange Zeit zwischenspeichern muß, was zu einem "Überlaufen" des absorbierenden Artikels und zu einer Rücknässung und/oder zu einer Beschmutzung der Bekleidung des Trägers führt.

Bei einer zu offenen zweiten Schicht besteht die Gefahr, daß die anfallende Flüssigkeitsmenge nicht komplett über die Breite des flüssigkeitsspeichemden Kernes verteilt wird, und somit dessen Kapazität nicht optimal ausgenutzt wird.
Diese Aufnahme- und Verteilungsschicht für Flüssigkeiten besteht aus einem Vliesstoff mit einer Längenausdehnung in x-Richtung, einer Breitenausdehnung in y-Richtung und einer Ausdehnung in die Tiefe in z-Richtung. Der Vliesstoff besitzt eine Ober- und eine Unterseite, wobei die Oberseite in Richtung körperseitiger Abdeckung weist, und die Unterseite in Richtung des flüssigkeitsspeichernden Kernes zeigt.

Es gibt Vliesstoffe welche aus einer Mischung von Stapelfasern und Mikrofasern besehen.
Derartige Vliesstoffe sind beispielsweise aus der US-Patentschrift 4,100,324 oder aus der deutschen Patentschrift DE 2735063 bekannt. Die vorgenannten Schriften gehen im wesentlichen von einem homogenen Verhältnis der Mischung zwischen Mikrofasern und Stapelfasern in z-Richtung aus.

Es bestand die Aufgabe, eine Aufnahme- und Verteilungselement für Flüssigkeiten in absorbierenden Artikeln zu entwickeln, welches die vorgenannten Nachteile nicht zeigt und eine gute Steuerbarkeit der Flüssigkeitsaufnahme- und Verteilungsfunktion bietet.

Die Aufgabe wird gemäß den Merkmalen des Anspruchs 1 gelöst. Vorteilhafte Ausgestaltung der Erfindung werden in den Unteransprüchen beschrieben. Die besonderen Vorteile des erfindungsgemäßen Aufnahme- und Verteilungselementes werden dabei in der guten Steuerbarkeit des Verhältnisses zwischen Flüssigkeitsaufnahme- und Flüssigkeitsverteilungsfunktion gesehen.

### Erläuterung der Zeichnung:

Die Figur zeigt den Schnitt durch eine bevorzugte Ausführungsform eines absorbierenden Artikels, welcher das erfindungsgemäße Aufnahme- und Verteilungselement beinhaltet.

Im Gegensatz zum Stand der Technik weist der Vliesstoff des erfindungsgemäßen Aufnahme- und Verteilungselementes auf seiner Oberseite eine Anreicherung der in der Mischung enthaltenen Stapelfasern auf, wohingegen die Unterseite des Vliesstoffes eine Anreicherung der in der Mischung vorhandenen Mikrofasern aufweist. Desweiteren besteht zwischen Oberseite und Unterseite in der z-Richtung des Vliesstoffes ein Gradient im Fasermischungsverhältnis zwischen Stapelfasern und Mikrofasem und demzufolge auch ein Gradient in der Porosität sowie im mittleren Faserdurchmesser.

Die Mikrofasern des erfindungsgemäßen Aufnahme- und Verteilungselementes können aus jedem thermoplastischen Kunststoff bestehen. Beispielsweise sind dies Kunststoffe aus der Reihe der Polyester, Polyamide, der Polystyrole, der Polycarbonate oder der Polyolefine, von denen Kunststoffe aus Polypropylen, aus Polyethylen oder aus copolymeren Polyolefinen besonders bevorzugt sind. Die Faserdurchmesser, welche bei den Mikrofasem vorliegen, betragen zwischen 0,5 und 20 µ, bevorzugt 1,0 bis 10,0 µ.
In einer bevorzugten Ausführungsform liegen die Mikrofasem in einer hydrophilen Ausführung vor, die Lehre zur Herstellung hydrophiler Mikrofasern ist z.B den US-Patentschriften 4,307,143 und 4,578,414 zu entnehmen. In einer alternativen Ausführungsform kann der Vliesstoff mittels eines, dem Fachmann geläufigen Ausrüstverfahrens z.B. mittels einer Sprüh-, Pflatsch- oder Tränktechnik mit einer hydrophilen Ausrüstung versehen werden.

Bei den Stapelfasern des erfindungsgemäßen Aufnahme- und Verteilungselementes handelt es sich um gekräuselte Fasern beispielsweise mit einer Faserlänge von 5 bis 100 mm und einem Fasertiter von 0,8 bis 30 dtex. Als Stapelfaser sind zu 100 % oder unter sich in Mischung alle in einem textilen Prozeß verarbeitbare Stapelfasern geeignet (z.B. aus Polyester, Polypropylen, Polyamid, Polyacrylnitril, Viskosefasem, Hanffasern, Jutefasern, Flachsfasern, Sisalfasern, Kenaffasern, Kapokfasem). Weiterhin sind Zellulose-Pulpe-Fasem zu 100 % oder in Mischung mit den vorstehend genannten Fasern geeignet.

In einer bevorzugten Ausführungsform sind den Stapelfasern Bindefasem zugemischt, so daß die Stapelfasern aus Matrixfasern und Bindefasern zusammengesetzt sind. Als Matrixfasern kommen die vorgenannten Fasertypen in Frage, als Bindefasern werden bevorzugt Mantel-Kern-Fasern eingesetzt, mit einem Kern aus Polyester und einem Mantel aus Co-Polyester oder einem Kern aus Polyester und einem Mantel aus Polyethylen oder mit einem Kern aus Polypropylen und einem Mantel aus Polyethylen.
Der Anteil der Matrixfasern in den Stapelfasern kann 5% bis 100% betragen, der Anteil der Bindefasern kann zwischen 95% und 0% betragen.
Im Falle der Zumischung von Bindefasern ist eine thermische Verfestigung des Vliesstoffes sinnvoll. Die Verfestigung erfolgt mittels heißer Kalanderwalzen oder mittels Heißluft, bei einer Temperatur, welche oberhalb des Schmelzpunktes des Mantels der Bindefasern, aber unterhalb des Schmelzpunktes des Kernes der Bindefasern und des Schmelzpunktes der Matrixfasern liegt. Eine derartige thermische Verfestigung bewirkt eine erhöhte Zugfestigkeit sowie eine erhöhte Druckstabilität des Vliesstoffes.

Der Vliesstoff des erfindungsgemäßen Aufnahme- und Verteilungselementes besitzt eine Flächenmasse von 5 bis 100 g/m² wobei eine Flächenmasse von 10 bis 50 g/m² bevorzugt wird.

Die Dicke des erfindungsgemäßen Aufnahme- und Verteilungselementes beträgt gemessen nach EDANA 30.5-99 Abschn. 5.3 Methode B (EDANA = European Disposables and Nonwovens Association, mit Sitz Brüssel) 0,3 bis 3 mm, bevorzugt 0,5 bis 2 mm.

Wie bereits erwähnt, sind im Vliesstoff die Stapelfasern mit den Mikrofasern derart gemischt, daß auf der Oberfläche des Vliesstoffes eine Anreicherung von Stapelfasern vorliegt und auf der Unterseite des Vliesstoffes eine Anreicherung von Mikrofasern vorliegt.
Zwischen Ober- und Unterseite des Vliesstoffes in z-Richtung des Vliesstoffes, befindet sich ein Gradient des Mischungsverhältnisses zwischen Stapelfasern und PP-Fasern.

Insgesamt betrachtet, kann das Verhältnis von Stapelfasern zu Mikrofasem von 5 : 95 bis 95 : 5 Gewichtsprozent, bevorzugt von 30 :70 bis 70:30 Prozent variieren.

Innerhalb der z-Richtung des Vliesstoffes befindet sich oberseitig eine Anreicherung von Stapelfasern, unterseitig eine Anreicherung von Mikrofasern. Dies bedeutet, daß oberseitig zwar in der Mehrzahl Stapelfasern vorliegen können, diese aber trotzdem mehr oder weniger mit Mikrofasern durchmischt sein können. Das gleiche gilt für die Unterseite, wo überwiegend Mikrofasern angereichert sind, diese aber trotzdem mehr oder weniger mit Stapelfasern durchmischt sein können.

Dadurch, daß die Oberseite eine Anreicherung von Stapelfasern besitzt, welche einen wesentlich höheren Faserdurchmesser besitzen als die Mikrofaser, und zudem noch gekräuselt sind, weist die Oberseite eine größere Porosität auf als die Unterseite des Vliesstoffes.
Die sich auf der Unterseite befindliche Anreicherung der Mikrofasem besitzt eine wesentlich niedrigere Porosität als die Oberseite. Das Mischungsverhältnis zwischen Stapelfasern und Mikrofasern in z-Richtung des Vliesstoffes ändert sich kontinuierlich, und bildet in z-Richtung einen Porositätsgradienten.

Das gleiche gilt für den mittleren Faserdurchmesser, welcher aus den unterschiedlichen Anteilen der beteiligten Fasern berechnet wird.
Auch für den für den mittleren Faserdurchmesser bildet sich in z-Richtung ein Gradient aus, wobei der mittleren Faserdurchmesser an der Seite der Anreicherung der Stapelfasern größer ist, als an der Seite der Anreicherung der Mikrofasern.

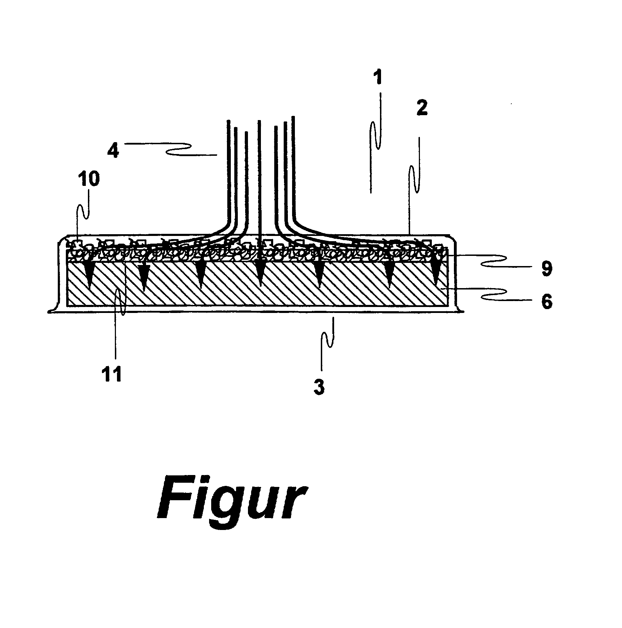

Die Figur zeigt im Schnitt einen absorbierenden Einwegartikel **1** bestehend aus einer körperseitigen Abdeckung **2**, einer bekleidungsseitigen Abdeckung **3**, einem flüssigkeitsspeichernden Kern **6** und zwischen dem flüssigkeitsspeichernden Kern **6** und der körperseitigen Abdeckung **2** angeordnet, das erfindungsgemäße Aufnahmeund Verteilungselement **9**.
Aus der Figur 1 ist ersichtlich, daß der auftreffende Flüssigkeitsschwall **4** eine Verteilung der Flüssigkeit über die Flächen des absorbierenden Kernes erfährt. An der Oberseite **10** des erfindungsgemäßen Aufnahme- und Verteilungselementes **9** befindet sich eine Anreicherung von Stapelfasern und damit eine höhere Porosität als an der Unterseite **11** wo eine Anreicherung der Mikrofasem vorliegt. Das erfindungsgemäßen Aufnahme- und Verteilungselementes **9** bewirkt, daß der auftreffende Flüssigkeitsschwall **4** an der Unterseite des erfindungsgemäßen Aufnahme- und Verteilungselementes **9** leicht zurückstaut und somit auf die Fläche des flüssigkeitsspeichernden Kernes **6** verteilt wird. Die Oberseite **10** des Aufnahmeund Verteilungselementes **9** wirkt hier als Zwischenspeicher der auftreffenden Flüssigkeit und verhindert, daß die großen anfallenden Flüssigkeitsmengen wieder zurück an den Körper gelangen.

Durch Variation des Gradienten der mittleren Faserdurchmessers in z-Richtung des Aufnahme- und Verteilungselementes **9**, läßt sich diese Rückstau- und Speichereigenschaft in optimaler Weise steuern. Verstärkt man die Anreicherung der Stapelfasern an der Oberfläche **10** wird die Zwischenspeicher- und die Verteileigenschaft des erfindungsgemäßen Aufnahme- und Verteilungselementes **9** erhöht und die Eindringzeit der Flüssigkeit in den flüssigkeitsspeichemden Kern verlängert.
Verringert man dagegen die Anreicherung der Stapelfasern an der Oberfläche **10** wird die Eindringzeit der Flüssigkeit in den flüssigkeitsspeichernden Kern verkürzt und die Zwischenspeicher- und die Verteileigenschaft erniedrigt. Damit ist es dem Fachmann möglich, das Verhältnis zwischen Zwischenspeicher- und die Verteileigenschaft des erfindungsgemäßen Aufnahme- und Verteilungselementes **9** einerseits, und der Eindringzeit der Flüssigkeit in den flüssigkeitsspeichernden Kern andererseits, exakt einzustellen.

In einer bevorzugten Ausführungsform kann das erfindungsgemäße Aufnahme- und Verteilungselement **9** mit Perforationen versehen sein. Diese Perforationen können beispielsweise nach dem in der DE 3416004 oder nach dem im DE 29720192 beschriebenen Verfahren hergestellt werden. Derartige Perforationen sind beispielsweise dann sinnvoll, wenn zusätzlich zu flüssigen Körperausscheidungen niedrig- bis mittelviskose Körperausscheidungen in den absorbierenden Artikel eingelagert werden sollen. Die Perforationen führen zu einem direkten Kontakt der niedrig- bis mittelviskosen Körperausscheidungen mit dem flüssigkeitsspeichernden Kern, wobei diese gespeichert und durch schnelle Entwässerung immobilisiert werden. Die nicht perforierten Stellen wirken dagegen in der vorher beschriebenen Weise. Geeignete Perforationen haben einen Durchmesser von mindestens 0,5 mm, bei einer Dichte von mindestens 0,5 Perforationen pro cm².

In einer weiteren bevorzugten Ausführungsform kann das erfindungsgemäße Aufnahme- und Verteilungselement **9** mit einer Trägerschicht versehen sein, etwa um die Stabilität des erfindungsgemäßen Aufnahme- und Verteilungselementes **9** zu erhöhen um z.B. verbesserte Verarbeitungseigenschaften zu erreichen. Die Trägerschicht kann aus einem kalanderverfestigten Vliesstoff, einem Spinnvlies, oder einem wasserstrahlverfestigten Vliesstoff bestehen.

### Beispiel: Zur Erläuterung des Porositätsgradienten wurde folgender Versuch durchgeführt:

a) Meßmethode: Die Porosität wird in vorliegendem Beispiel durch das Merkmal der Luftdurchlässigkeit charakterisiert, da davon ausgegangen wird, daß ein Stoff mit größeren Poren eine höhere Luftdurchlässigkeit besitzt, als ein Stoff mit kleineren Poren.
Desweiteren wird davon ausgegangen, daß ein Stoff mit einer höheren Luftdurchlässigkeit auch eine höhere Flüssigkeitsdurchlässigkeit besitzt. Die Luftdurchlässigkeit wurde in diesem Beispiel nach den Vorschriften der DIN 53887 bei einem Differenzdruck von 200 Pa und einer Meßfläche von 25 cm² bestimmt
b) Probenvorbereitung: Da es sich beim Porositätsgradienten um eine kontinuierliche Veränderung der Porosität in z-Richtung handelt, war es nicht möglich, diese an jeder Stelle in z-Richtung zu bestimmen. Ersatzweise wurde die Probe in z-Richtung in drei Teile gleicher Dicke gespalten, so daß sich drei Spalte, nämlich ein der Oberseite zugeordneter Spalt Sₒ, ein mittlerer Spalt Sₘ und ein der Unterseite zugeordneter Spalt Sᵤ ergaben von denen jeweils die Luftdurchlässigkeit bestimmt wurde.
c) Probe: (Vliesstoff des erfindungsgemäßen Aufnahme- und Verteilungselementes)

| | |
|---|---|
| Flächenmasse | 60 g/m² |
| Dicke | 2,4 mm |
| Verhältnis Stapelfasern zu Mikrofasern | 40 Gew. % zu 60 Gew. % |
| Mikrofaserdurchmesser | durchschnittlich 3,4 µ |
| Stapelfasertiter | 6,7 dtex |
| Material der Mikrofasern | Polypropylen |
| Material der Stapelfasern | Polyester gekräuselt |

d) Meßergebnisse:

| | |
|---|---|
| Luftdurchlässigkeit | Sₒ = 1980 I/(m² x sec) |
| | Sₘ = 1220 I/(m² x sec) |
| | Sᵤ = 810 I/(m² x sec) |

e) Schlußfolgerung: Aus den Meßergebnissen geht hervor, daß die Porosität der Probe von deren Oberseite zur Unterseite hin abnimmt.

## Patentansprüche

1. Aufnahme- und Verteilungselement für Flüssigkeiten in absorbierenden Artikeln, wie Babywindeln, Inkontinenzhilfen, Damenbinden und dergleichen, bestehend aus einem Vliesstoff, bei welchem Mikrofasern mit Stapelfasern vermischt sind, mit einer flächigen Ausdehnung in x-y-Richtung und einer in die Tiefe des Vliesstoffes zeigenden Ausdehnung in z-Richtung, mit einer zur körperseitigen Abdeckung zeigenden Oberseite und einer zum flüssigkeitsspeichernden Kern zeigenden Unterseite und wobei die Oberseite eine höhere Porosität und einen größeren durchschnittlichen Faserdurchmesser besitzt als die Unterseite
**dadurch gekennzeichnet,**
**daß** die Oberseite einen höheren Anteil Stapelfasern aufweist als die Unterseite, und die Unterseite einen höheren Anteil an Mikrofasern aufweist als die Oberseite, daß die Porosität von der Oberseite zur Unterseite kontinuierlich abnimmt und daß sich das Fasermischungsverhältnis zwischen Stapelfasern und Mikrofasern von der Oberseite zur Unterseite kontinuierlich verändert.

2. Aufnahme- und Verteilungselement nach Anspruch 1
**dadurch gekennzeichnet,**
**daß** die Mikrofasern des Vliesstoffes hydrophil sind.

3. Aufnahme- und Verteilungselement nach Anspruch 1
**dadurch gekennzeichnet,**
**daß** der Vliesstoff Perforationen aufweist.

4. Aufnahme- und Verteilungselement nach Anspruch 3
**dadurch gekennzeichnet,**
**daß** die Perforationen einen Durchmesser von mindestens 0,5 mm und eine Dichte von mindestens 0,5 Perforationen/cm² aufweisen.

5. Aufnahme- und Verteilungselement nach Anspruch 1
**dadurch gekennzeichnet,**
**daß** der Vliesstoff auf der Unterseite eine Trägerschicht, bestehend aus einem kalanderverfestigten Vliesstoff, einem Spinnvlies oder einem wasserstrahlverfestigten Vliesstoff besitzt.

6. Aufnahme- und Verteilungselement nach Anspruch 1
**dadurch gekennzeichnet,**
**daß** der Vliesstoff eine Flächenmasse von 5 - 100 g/m² aufweist.

7. Aufnahme- und Verteilungselement nach Anspruch 1
**dadurch gekennzeichnet,**
**daß** die Stapelfasern einen Fasertiter von 0,8 bis 30 dtex aufweisen.

8. Aufnahme- und Verteilungselement nach Anspruch 7
**dadurch gekennzeichnet,**
**daß** die Stapelfasern aus 5 % bis 100% Matrixfasern und 95% bis 0 % Bindefasern bestehen.

9. Aufnahme- und Verteilungselement nach Anspruch 8
**dadurch gekennzeichnet,**
**daß** die Matrixfasern aus Polyester, Polypropylen, Polyethylen, Polyamid, Polyacrylnitril, Viskose, oder aus Naturfasern wie Cellulose-Pulp, Hanf, Jute, Flachs, Sisal, Baumwolle, Kenaf, Kapok, bestehen.

10. Aufnahme- und Verteilungselement nach Anspruch 9
**dadurch gekennzeichnet,**
**daß** es sich bei den Bindefasern um Mantel-Kern-Fasern mit einem Kern aus Polyester und einem Mantel aus Co-polyester, oder mit einem Kern aus Polyester und einem Mantel aus Polyethylen oder mit einem Kern aus Polypropylen und einem Mantel aus Polyethylen handelt.

## Claims

1. Absorption and distribution element for liquids in absorbent items, such as babies' napkins, incontinence pads, sanitary towels and the like, consisting of a bonded-fibre fabric in which microfibres are mixed with staple fibres, with a 2-dimensional expansion in the x-y direction and expansion in the z direction into the depth of the bonded-fibre fabric, with a body-facing upper surface and a lower surface facing the core that stores the fluid and in which the upper surface has a higher porosity and a higher average fibre diameter than the lower surface,
**characterised in that**
the upper surface has a higher number of staple fibres than the lower surface, and the lower surface has a higher number of microfibres than the upper surface, **characterised in that** the porosity decreases continuously from the upper surface to the lower surface, and **characterised in that** the fibre-blend ratio changes continuously between staple fibres and microfibres from the upper surface to the lower surface.

2. Absorption and distribution element in accordance with Claim 1,
**characterised in that**
the microfibres of the bonded-fibre fabric are hydrophilic.

3. Absorption and distribution element in accordance with Claim 1,
**characterised in that**
the bonded-fibre fabric is perforated.

4. Absorption and distribution element in accordance with Claim 3,
**characterised in that**
the perforations have a diameter of at least 0.5 mm and a density of at least 0.5 perforations/cm².

5. Absorption and distribution element in accordance with Claim 1,
**characterised in that**
the bonded-fibre fabric on the lower surface has a support layer, consisting of a calender-reinforced bonded-fibre fabric, a woven fabric or a-water-jet-reinforced bonded-fibre fabric.

6. Absorption and distribution element in accordance with Claim 1,
**characterised in that**
the bonded-fibre fabric has a superficial mass of 5 - 100 g/m².

7. Absorption and distribution element in accordance with Claim 1,
**characterised in that**
the staple fibres have a fibre titre of between 0.8 and 30 dtex.

8. Absorption and distribution element in accordance with Claim 7,
**characterised in that**
the staple fibres are comprised of 5% to 100% matrix fibres and 95% to 0% binding fibres.

9. Absorption and distribution element in accordance with Claim 8,
**characterised in that**
the matrix fibres are comprised of polyester, polypropylene, polyethylene, polyamide, polyacrylnitrile, viscose, or of natural fibres such as cellulose pulp, hemp, jute, flax, sisal, cotton, kenaf and kapok.

10. Absorption and distribution element in accordance with Claim 8,
**characterised in that**
the binding fibres comprise the mantle-core fibres with a polyester core and a co-polyester mantle, or with a polyester core and a polyethylene mantle or with a polypropylene core and a polyethylene mantle.

## Revendications

1. Elément de réception et de dispersion de liquidés dans les articles absorbants, telles que les couches pour bébé, les protections contre l'incontinence, les serviettes hygiéniques, etc., composé d'un non-tissé, dans lequel des microfibres sont mélangées avec des fibres coupées, avec une extension dans le sens x-y et une extension orientée dans la profondeur du non-tissé dans le sens z, avec une face supérieure orientée vers une couverture côté corps et une face inférieure orientée vers le coeur retenant le liquide, et où la face supérieure possède une porosité élevée et un diamètre de fibres moyen plus grand que la face inférieure
**caractérisé par le fait que**
la face supérieure présente une proportion de fibres coupées plus élevée que la face inférieure, et la face inférieure présente une proportion de microfibres plus élevée que la face supérieure, que la porosité diminue régulièrement de la face supérieure vers la face inférieure et que le rapport de mélange des fibres entre les fibres coupées et les microfibres change régulièrement de la face supérieure vers la face inférieure.

2. Elément de réception et de dispersion selon la revendication 1
**caractérisé par le fait que**
les microfibres du non-tissé sont hydrophiles.

3. Elément de réception et de dispersion selon la revendication 1
**caractérisé par le fait que**
le non-tissé présente des perforations.

4. Elément de réception et de dispersion selon la revendication 3
**caractérisé par le fait que**
les perforations présentent un diamètre d'au moins 0,5 mm et une densité d'au moins 0,5 perforations/cm2.

5. Elément de réception et de dispersion selon la revendication 1
**caractérisé par le fait que**
le non-tissé possède une couche support sur la face inférieure, composée d'un non-tissé consolidé par calandrage, d'un non-tissé spunlace ou d'un non-tissé consolidé par jet d'eau.

6. Elément de réception et de dispersion selon la revendication 1
**caractérisé par le fait que**
le non-tissé présente un grammage de 5 - 100 g/m2.

7. Elément de réception et de dispersion selon la revendication 1
**caractérisé par le fait que**
les fibres coupées présentent un titre compris entre 0,8 et 30 dtex.

8. Elément de réception et de dispersion selon la revendication 7
**caractérisé par le fait que**
les fibres coupées sont composées entre 5 % et 100 % de fibres matricielles et entre 95 % et 0 % de fibres de liage.

9. Elément de réception et de dispersion selon la revendication 8
**caractérisé par le fait que**
les fibres matricielles sont en polyester, polypropylène, polyéthylène, polyamide, polyacrylnitrile, viscose ou en fibres naturelles comme la pâte de cellulose, le chanvre, le jute, le lin, le sisal, le coton, le kénaf, le kapok.

10. Elément d'absorption et de dispersion selon la revendication 8
**caractérisé par le fait**
**qu'**il s'agit de fibres gaine-noyau pour les fibres de liage avec un noyau en polyester et une gaine en co-polyester, ou avec un noyau en polyester et une gaine en polyéthylène ou avec un noyau en polypropylène et une gaine en polyéthylène.
